(19) Europäisches Patentamt
European Patent Office
Office européen des brevets

(11) Veröffentlichungsnummer: **0 094 038**
**B1**

(12) # EUROPÄISCHE PATENTSCHRIFT

(45) Veröffentlichungstag der Patentschrift: **07.01.87**

(51) Int. Cl.[4]: **C 07 D 487/04**, A 61 K 31/50, A 61 K 31/41

(21) Anmeldenummer: **83104427.6**

(22) Anmeldetag: **05.05.83**

(54) **3-Amino-6-aryl-1,2,4-triazolo(4,3-b)-pyridazine, ihre Herstellung und ihre Verwendung.**

(30) Priorität: **08.05.82 DE 3217325**

(43) Veröffentlichungstag der Anmeldung: **16.11.83 Patentblatt 83/46**

(45) Bekanntmachung des Hinweises auf die Patenterteilung: **07.01.87 Patentblatt 87/2**

(84) Benannte Vertragsstaaten: **AT BE CH DE FR GB IT LI LU NL SE**

(56) Entgegenhaltungen:
**EP-A-0 029 103**
**DE-A-1 670 095**
**DE-A-2 113 438**
**DE-A-2 741 763**
**US-A-3 708 484**

(73) Patentinhaber: **HOECHST AKTIENGESELLSCHAFT, Postfach 80 03 20, D-6230 Frankfurt am Main 80 (DE)**

(72) Erfinder: **Rösner, Manfred, Dr., Unter den Buchen 7, D-6239 Eppstein/Taunus (DE)**
Erfinder: **Hock, Franz, Dr., Altstadt 19, D-6110 Dieburg (DE)**

EP 0 094 038 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es wurde schon beschrieben, dass Verbindungen, welche das Triazolo[4,3-b]-pyridazin-Ringsystem besitzen, pharmakologisch wirksam sind. So wird für die in der DE-A 2 113 438 erwähnten Nitrofuryl-triazolo[4,3-b]pyridazin-Derivate eine antimikrobielle Wirksamkeit angegeben. Die 6-Phenyl-1,2,4-triazolo[4,3-b]pyridazine entsprechend DE-A 2 741 763 und die 6- und 8-Heteroaryl-1,2,4-triazolo[4,3-b]pyridazine entsprechend EP-B 0 029 103, welche in 3-Stellung ein Wasserstoffatom oder eine $C_1$–$C_3$-Alkylgruppe tragen, besitzen anxiolytische Wirksamkeit, wobei die Verbindungen gemäss DE-A 2 741 763 zusätzlich noch blutdrucksenkend wirken. Schliesslich werden in der US-Patentschrift 3 708 484 3-Amino-triazolo[4,3-b]-pyridazin-Derivate, welche in 7- oder 8-Stellung eine weitere basische Gruppierung tragen, mit antidepressiver Wirkung beschrieben. Zusätzlich wird noch eine antiinflammatorische Aktivität angegeben.

Es wurde nun überraschenderweise gefunden, dass 6-Aryl-1,2,4-triazolo[4,3-b]-pyridazine, welche in 3-Stellung durch eine Aminogruppe substituiert sind, anxiolytische und anticonvulsive Eigenschaften, welche nicht mit blutdrucksenkenden einhergehen, besitzen.

Die Erfindung betrifft daher neue 3-Amino-6-aryl-1,2,4-triazolo[4,3-b]pyridazine der allgemeinen Formel I

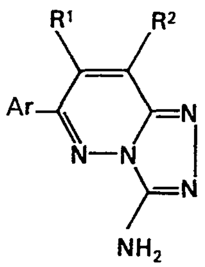
I

und deren Salze mit einer physiologisch verträglichen Säure, worin
$R^1$ und $R^2$ gleich oder verschieden sind und Wasserstoff, Alkylgruppen mit 1-6 C-Atomen, Phenyl oder Chlor darstellen und Ar für Phenyl, Biphenyl, Phenoxyphenyl, Phenylthiophenyl, Phenylsulfinylphenyl, Phenylsulfonylphenyl, 1- oder 2-Naphthyl, 2- oder 3-Thienyl, 2-Furyl, 2-Pyrrolyl, 1-Methyl-2-pyrrolyl, 2-, 3- oder 4-Pyridyl steht, wobei diese Reste gegebenenfalls durch Fluor, Chlor, Brom, Jod, Aklylgruppen mit 1-6 C-Atomen, Cycloalkylgruppen mit 3-8 C-Atomen, Phenylalkylgruppen mit 1-4 Alkyl-C-Atomen, Alkoxy oder Alkylthiogruppen mit jeweils 1-6 C-Atomen, Hydroxy, Nitro, Cyano, Trifluormethyl, Carboxygruppen, deren Ester mit $C_1$–$C_6$-Alkoholen, Aminocarbonyl, Amino, Acetamino, Alkoxycarbonylamino mit 1-6 C-Atomen im Alkylrest ein-, zwei-, drei-, vier- oder fünffach substituiert sein können.

Unter den Verbindungen der allgemeinen Formel I sind solche bevorzugt, in denen $R^1$ und $R^2$ gleich oder verschieden sind und Wasserstoff, Methyl, Ethyl, Phenyl oder Chlor bedeuten und in denen Ar Phenyl, Biphenyl, 2- oder 3-Thienyl, 2-Furyl, 2-, 3- oder 4-Pyridyl bedeutet, die gegebenenfalls durch ein, zwei oder drei Fluor, Chlor, Brom, Alkylgruppen mit 1-6 C-Atomen, Cycloalkylgruppen mit 3-6 C-Atomen oder Trifluormethyl substituiert sein können.

Besonders bevorzugt sind solche Verbindungen der Formel I, in denen $R^1$ und $R^2$ gleich oder verschieden sind und Wasserstoff, Methyl oder Ethyl und Ar Phenyl, 2- oder 3-Thienyl, 2-, 3- oder 4-Pyridyl, gegebenenfalls durch ein oder zwei Fluor, Chlor, Methyl, Ethyl oder Trifluormethyl substituiert, bedeuten.

Gegenstand der Erfindung sind auch Verfahren zur Herstellung dieser Verbindungen sowie pharmazeutische Zubereitungen dieser Verbindungen und ihre Verwendung als Arzneimittel.

Das Verfahren zur Herstellung von Verbindungen der Formel I ist dadurch gekennzeichnet, dass man

a) ein Aryl-hydrazino-pyridazin der Formel II

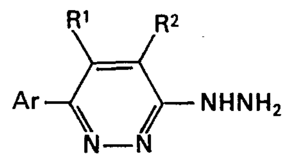
II

worin $R^1$, $R^2$ und Ar die zu Formel I angegebenen Bedeutungen haben, mit Chlorcyan, Bromcyan, O-Methylisoharnstoff oder S-Methylisothioharnstoff, Guanidin oder deren Salze, Chlorformamidinhydrochlorid oder Cyanamid als Cyclisierungsreagenz umsetzt, oder

ein Aryl-hydrazinopyridazin der Formel II

b) mit einem N-($R^3$-Oxy)-carbonyl-O-methylisoharnstoff zu einer Verbindung der Formel III

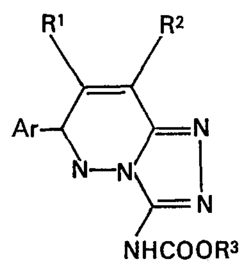
III

worin Ar, $R^1$ und $R^2$ die zu Formel I angegebenen Bedeutungen haben und $R^3$ Alkyl mit 1–10 C-Atomen, Benzyl oder Phenyl bedeutet, umsetzt und anschliessend die so erhaltene Verbindung verseift, oder

c) eine Verbindung der Formel IV, worin $R^4$ Chlor, Brom

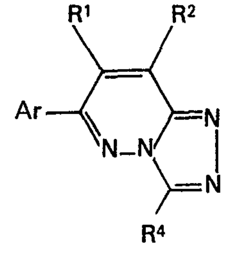
IV

oder Methylthio bedeutet, und Ar, $R^1$ und $R^2$ die zu Formel I angegebenen Bedeutungen haben, mit Ammoniak umsetzt, oder

d) eine Verbindung der Formel V

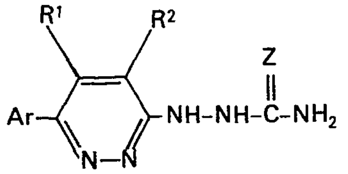

V

worin Ar, $R^1$ und $R^2$ die zu Formel I angegebenen Bedeutungen haben und Z für O, S oder NH steht, durch Erhitzen, gegebenenfalls unter Zusatz eines Kondensationsmittels, zu einer Verbindung der Formel I cyclisiert. Verbindungen der Formel II sind z.B. aus J. Heterocyclic Chem. 15, 881 (1978) bekannt und können aus Chlorverbindungen der Formel VI mit Hydrazinhydrat

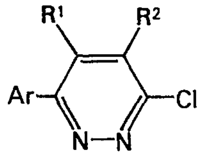

VI

nach literaturbekannten Verfahren (The Chemistry of Heterocyclic Compounds, Vol. 28 Pyridazines, Editors A. Weissberger und E.C. Taylor, John Wiley, New York 1973) hergestellt werden. Beide Literaturstellen beschreiben auch die Synthese der Chlorverbindungen VI und ihrer Vorstufen.

Das Verfahren a) wird in einem geeigneten Lösungsmittel in einem Temperaturbereich von 0°C bis zum Siedepunkt des verwendeten Lösungsmittels durchgeführt, vorzugsweise bei 50 bis 100°C.

Als Lösungsmittel für das Verfahren a) kommen z.B. in Frage Wasser, Essigsäure, aliphatische Alkohole wie Methanol, Ethanol, Isopropanol, Dioxan, DMF, Toluol, chlorierte Kohlenwasserstoffe wie Methylenchlorid, Chloroform, Dichlorethan, Tetrachlorkohlenstoff.

Das Verfahren b) wird in einem geeigneten Lösungsmittel in Gegenwart einer Säure durchgeführt. Beispielsweise arbeitet man in einem Gemisch Methanol/Eisessig in einem Volumenverhältnis von z.B. 10:1 bei Rückflusstemperatur. Die Verseifung des Carbaminats III erfolgt unter alkalischen Bedingungen durch Erhitzen mit einem Alkali- oder Erdalkalimetallhydroxid wie z.B. NaOH, KOH, $Ca(OH)_2$, $Ba(OH)_2$ in einem Lösungsmittel oder Lösungsmittelgemisch auf 60–180°C vorzugsweise auf 100–140°C. Als Lösungsmittel kommen insbesondere Wasser und aliphatische Alkohole und Diole wie Ethanol, Propanol, Isopropanol, Butanol, 2-Methoxyethanol, Glykol in Frage.

Für das Verfahren c) werden die Ausgangsstoffe der Formel IV durch Erhitzen von Arylhydrazinopyridazinen II mit Ameisensäure oder deren Estern (bei $R^4$ = H), Chlorameisensäureestern oder einem Dialkylcarbonat (bei $R^4$ = OH) oder mit Schwefelkohlenstoff und Alkali (bei $R^4$ = SH), gegebenenfalls unter Zusatz eines Lösungs- oder Verdünnungsmittels wie Chloroform, Toluol, Dioxan, Wasser, Ethanol, zu einer Verbindung der Formel IV′ umgesetzt, in der $R^4$ = H, OH oder SH bedeutet. Durch Erhitzen mit Brom in Eisessig/Natriumacetat (bei $R^4$ = H), Phosphoroxychlorid (bei $R^4$ = OH) bzw. Methyljodid oder Dimethylsulfat (bei $R^4$ = SH) werden daraus die entsprechenden Verbindungen IV mit $R^4$ = Br, Cl, $SCH_3$ erhalten.

Nach Verfahren c) erfolgt die Umsetzung der Verbindungen der Formel IV mit flüssigem, wässrigem oder alkoholischem Ammoniak, oder in einem inerten Lösungsmittel wie Methanol, Dioxan oder Toluol mit gasförmigem Ammoniak durch Einleiten, oder unter Druck. Der Temperaturbereich erstreckt sich von der Temperatur des flüssigen Ammoniaks bis zu 200°C.

Nach Verfahren d) werden die z.B. durch Umsetzung von Chlorverbindungen VI mit Semicarbazid, Thiosemicarbazid oder Aminoguanidin in einem Lösungsmittel wie z.B. Methanol, Ethanol, Isopropanol, DMF, Tetrahydrofuran, Toluol, Chloroform oder Dichlorethan erhaltenen Verbindungen V durch Erhitzen, z.B. in einem der genannten Lösungsmittel auf 40–150°C, gegebenenfalls unter Zusatz eines Kondensationsmittels wie Eisessig, Cyclohexylcarbodiimid, 1-Hydroxybenztriazol, in Verbindungen der Formel I übergeführt.

Wenn die Verbindungen der Formel I nach den beschriebenen Verfahren als Salze erhalten werden, so kann daraus mit Ammoniak, Aminen oder Hydroxiden die zugehörige Base freigesetzt werden.

Die freien Basen der Formel I werden mit physiologisch verträglichen Säuren in die entsprechenden Salze übergeführt. Als Säuren kommen anorganische oder organische Säuren in Betracht wie Chlor- oder Bromwasserstoffsäure, Phosphorsäure, Essigsäure, Benzoesäure, Citronensäure, Maleinsäure, Fumarsäure, Milchsäure, Weinsäure, Bernsteinsäure, Acetylglycin. Bevorzugt werden die Hydrochloride der Formel I.

Die erfindungsgemässen Verbindungen der Formel I sind zur Herstellung von Arzneimitteln geeignet. Die Arzneimittel können eine oder mehrere der erfindungsgemässen Verbindungen oder auch Mischungen derselben mit anderen pharmazeutisch wirksamen Stoffen enthalten. Zur Herstellung der Arzneimittel können die üblichen pharmazeutischen Träger- und Hilfsstoffe und bekannte galenische Verfahren verwendet werden. Die Arzneimittel können enteral, parenteral, oral oder perlingual angewendet werden. Beispielsweise kann die Verabreichung in Form von Tabletten, Kapseln, Pillen, Dragees, Zäpfchen, Gelees, Cremes, Puder, Liquida, Stäubepulver oder Aerosolen erfolgen. Als Liquida kommen zum Beispiel in Frage: Ölige oder wässrige Lösungen oder Suspensionen, Emulsionen, injizierbare wässrige Lösungen oder Suspensionen.

Die erfindungsgemässen Verbindungen können ausserdem als Zwischenprodukte zur Herstellung anderer Arzneimittel Verwendung finden.

Als erfindungsgemässe Verbindungen seien genannt:

3-Amino-6-(2-bromphenyl)-1,2,4-triazolo[4,3-b]pyridazin, 3-Amino-6-(2-trifluormethylphenyl)-1,2,4-triazolo[4,3-b]pyridazin, 3-Amino-6-(4-chlor-3-trifluormethylphenyl)-1,2,4-triazolo[4,3-b]pyridazin, 3-Amino-6-(3-ethylphenyl)-1,2,4-triazolo[4,3-b]pyridazin, 3-Amino-6-(2-methylphenyl)-1,2,4-triazolo[4,3-b]pyridazin, 3-Amino-6-(2-ethylphenyl)-1,2,4-triazolo[4,3-b]pyridazin, 3-Amino-6-(3-methoxyphenyl)-1,2,4-triazolo[4,3-b]pyridazin, 3-Amino-6-(4-propylthiophenyl)-1,2,4-triazolo[4,3-b]pyridazin, 3-Amino-6-(4-acetamidophenyl)-1,2,4-triazolo[4,3-b]pyridazin, 3-Amino-6-(4-aminophenyl)-1,2,4-triazolo[4,3-b] pyridazin, 3-Amino-6-(4-methoxycarbonylaminophenyl)-1,2,4-triazolo[4,3-b]pyridazin, 3-Amino-6-(4-nitrophenyl)-1,2,4-triazolo[4,3-b]pyridazin, 3-Amino-6-(3-nitrophenyl)-1,2,4-triazolo[4,3-b] pyridazin, 3-Amino-6-(2-nitrophenyl)-1,2,4-triazolo[4,3-b]pyridazin, 3-Amino-6-(4-cyanophenyl)-1,2,4-triazolo[4,3-b]pyridazin, 3-Amino-6-(3-cyanophenyl)-1,2,4-triazolo[4,3-b]pyridazin, 3-Amino-6-(2-cyanophenyl)-1,2,4-triazolo[4,3-b] pyridazin, 3-Amino-6-(4-phenylsulfinylphenyl)-1,2,4-triazolo[4,3-b]pyridazin, 3-Amino-6-(4-phenylsulfonylphenyl)-1,2,4-triazolo[4,3-b]pyridazin, 3-Amino-6-(4-hydroxyphenyl)-1,2,4-triazolo[4,3-b]pyridazin, 3-Amino-6-(3-hydroxyphenyl)-1,2,4-triazolo[4,3-b]pyridazin, 3-Amino-6-(3,4-dihydroxyphenyl)-1,2,4-triazolo[4,3-b]pyridazin, 3-Amino-6-(1-naphthyl)-1,2,4-triazolo[4,3-b]pyridazin, 3-Amino-6-(2-naphthyl)-1,2,4-triazolo[4,3-b]pyridazin, 3-Amino-6-(3-thienyl)-1,2,4-triazolo[4,3-b]pyridazin, 3-Amino-6-(4-chlor-2-thienyl)-1,2,4-triazolo[4,3-b]pyridazin, 3-Amino-6-(3-methyl-2-thienyl)-1,2,4-triazolo[4,3-b]pyridazin, 3-Amino-6-(4-methyl-2-thienyl)-1,2,4-triazolo[4,3-b]pyridazin, 3-Amino-6-(2-furyl)-1,2,4-triazolo[4,3-b]pyridazin, 3-Amino-6-(5-methyl-2-furyl)-1,2,4-triazolo[4,3-b]pyridazin, 3-Amino-6-(4-pyridyl)-1,2,4-triazolo[4,3-b]pyridazin, 3-Amino-6-(3-pyridyl)-1,2,4-triazolo[4,3-b]pyridazin, 3-Amino-6-(2-pyridyl)-1,2,4-triazolo[4,3-b]pyridazin, 3-Amino-6-(2-pyrrolyl)-1,2,4-triazolo[4,3-b]pyridazin und 3-Amino-6-(1-methyl-2-pyrrolyl)-1,2,4-triazolo [4,3-b]pyridazin.

Die erfindungsgemässen Verbindungen der Formel I besitzen pharmakologische Eigenschaften; insbesondere wirken sie anxiolytisch und antikonvulsiv. Als Indikationen kommen deshalb Schlaflosigkeit, Erregung und vegetative Verstimmung in Betracht.

Die pharmazeutischen Zubereitungen enthalten im allgemeinen zwischen 1 bis 10% der erfindungsgemässen aktiven Komponente(n).

Die anxiolytische Wirkung der Verbindungen der Formel I ist von einer sehr geringen Sedierung und guten Verträglichkeiten begleitet ($LD_{50}$ i.a. >300 mg/kg i.p. an der Maus). Dies geht aus Untersuchungen hervor, bei denen der Einfluss der erfindungsgemässen Verbindungen auf die motorische Aktivität, die Hexobarbital-Narkose und den Cardiazolkrampf bei Mäusen gemessen wurde. Ausserdem wurde noch der Geller-Anxiolysetest sowie der Lick-Shock-Test an Ratten herangezogen.

Die niedrigste bereits wirksame Dosis in den oben angegebenen Versuchen ist beispielsweise 5 mg/kg oral, 2,5 mg/kg sublingual, 1 mg/kg intravenös. Als allgemeiner Dosisbereich für Wirkung (Tierversuch wie oben) kommt beispielsweise in Frage: 5 bis 50 mg/kg oral, 2,5 bis 25 mg/kg sublingual, 1 bis 10 mg/kg intravenös.

Beispielsweise können 3 mal täglich 1 bis 3 Tabletten mit einem Gehalt von 10 bis 100 mg wirksamer Substanz oder zum Beispiel bei intravenöser Injektion 1 bis 3 mal täglich eine Ampulle von 2 bis 4 ml Inhalt mit 0,5 bis 5 mg Substanz appliziert werden.

Beispiel 1 Verfahren a)

3-Amino-6-phenyl-1,2,4-triazolo[4,3-b]pyridazin-hydrochlorid

25 g 3-Hydrazino-6-phenylpyridazin und 17,1 g Bromcyan werden in 150 ml Ethanol 3 Stunden unter Rückfluss gerührt und anschliessend im Vakuum einrotiert.

Der Rückstand wird in Wasser heiss gelöst, filtriert und mit überschüssigem Ammoniak alkalisch gestellt. Die freie Base wird abgesaugt, neutral gewaschen und getrocknet, in Ethanol suspendiert, mit ethanolischer Salzsäure versetzt und im Vakuum eingeengt.

Das ausgefallene Hydrochlorid wird abgesaugt, mit Ethanol gewaschen und getrocknet, Fp. 280°C (Zers.).

Beispiel 2 Verfahren a)

3-Amino-6-(4-fluorphenyl)-1,2,4-triazolo[4,3-b]pyridazin-hydrochlorid

In eine Lösung von 5 g 3-(4-Fluorphenyl-6-hydrazino-pyridazin in 50 ml Ethanol wird bei 0–10°C ein geringer Überschuss an Chlorcyan eingeleitet und bei Raumtemperatur bis zur vollständigen Umsetzung weitergerührt (DC-Kontrolle).

Das ausgefallene Hydrochlorid wird abgesaugt, mit Isopropanol gewaschen und getrocknet, Fp. 284°C (Zers.).

Beispiel 3 Verfahren b)

3-Amino-6-(3,4-dichlorphenyl)-1,2,4-triazolo[4,3-b]pyridazin

3 g 6-(3,4-Dichlorphenyl)-3-methoxycarbonylamino-1,2,4-triazolo[4,3-b]pyridazin und 3 g Kaliumhydroxid werden in 10 ml Wasser und 20 ml 2-Methoxyethanol 16 Stunden zum Rückfluss erhitzt.

Das ausgefallene Produkt wird abgesaugt, neutral gewaschen und aus Isopropanol umkristallisiert, Fp. 281°C.

Beispiel 4 Verfahren c)

6-(4-Methylphenyl)-1,2,4-triazolo[4,3-b]pyridazin

5 g 3-Hydrazino-6-(4-methylphenyl)-pyridazin

werden in 10 ml Ameisensäure 2 Stunden lang unter Rückfluss gerührt. Nach dem Abkühlen wird mit Wasser verdünnt und das ausfallende Produkt abgesaugt, neutral gewaschen und getrocknet, Fp. 186°C.

3-Brom-6-(4-methylphenyl)-1,2,4-triazolo[4,3-b]pyridazin

4 g 6-(4-Methylphenyl)-1,2,4-triazolo[4,3-b]pyridazin und 3 g Natriumacetat werden in 20 ml Eisessig suspendiert. 1 ml Brom in 5 ml Eisessig wird zugetropft und die Lösung 5 Stunden zum Rückfluss erhitzt. Man engt zur Trockne ein, versetzt mit Wasser und saugt das Produkt ab. Nach Umkristallisation aus Isopropanol und Trocknen, Fp. 211°C.

3-Amino-6-(4-methylphenyl)-1,2,4-triazolo[4,3-b]pyridazin

3 g 3-Brom-6-(4-methylphenyl)-1,2,4-triazolo[4,3-b]pyridazin werden mit 25 ml Methanol und 25 ml conc. Ammoniaklösung bei 100°C im Autoklaven behandelt. Nach dem Abkühlen wird mit Wasser verdünnt, abgesaugt, neutral gewaschen und getrocknet, Fp. 261°C.

Beispiel 5 Verfahren d)

3-Phenyl-6-thiosemicarbazidopyridazin

6 g 3-Chlor-6-phenylpyridazin und 6 g Thiosemicarbazid werden in 60 ml Ethanol 3 Stunden zum Rückfluss erhitzt. Man saugt den entstandenen Niederschlag ab, wäscht mit Ethanol und Wasser und trocknet im Vakuum, Fp. 213°C (Zers.).

3-Amino-6-phenyl-1,2,4-triazolo[4,3-b]pyridazin

2 g 3-Phenyl-6-thiosemicarbazidopyridazin werden in 20 ml Eisessig 6 Stunden lang zum Rückfluss erhitzt. Nach Einengen der Reaktionslösung wird mit wässrigem Ammoniak verrührt, abgesaugt, mit Wasser neutral gewaschen, getrocknet und aus Isopropanol umkristallisiert, Fp. 212°C.

Analog zu den Beispielen 1–5 können die folgenden Verbindungen hergestellt werden:

| Beispiel | Verbindung | Fp. | Salz | Verf. |
|---|---|---|---|---|
| 6. | 3-Amino-6-(4-bromphenyl)-1,2,4-triazolo[4,3-b]pyridazin | 317° (Zers.) | HBr | B |
| 7. | 3-Amino-6-(4-chlorphenyl)-1,2,4-triazolo[4,3-b]pyridazin | 264°C | – | C |
| 8. | 3-Amino-6-(4-jodphenyl)-1,2,4-triazolo[4,3-b]pyridazin | – | – | A |
| 9. | 3-Amino-6-(4-trifluormethylphenyl)-1,2,4-triazolo[4,3-b]pyridazin | 289°C (Zers.) | HCl | A |
| 10. | 3-Amino-6-(3-bromphenyl)-1,2,4-triazolo[4,3-b]pyridazin | 273°C (Zers.) | HCl | D |
| 11. | 3-Amino-6-(3-chlorphenyl)-1,2,4-triazolo[4,3-b]pyridazin | 288°C (Zers.) | HCl | A |
| 12. | 3-Amino-6-(3-fluorphenyl)-1,2,4-triazolo[4,3-b]pyridazin | 266°C (Zers.) | HCl | A |
| 13. | 3-Amino-6-(3-trifluormethylphenyl)-1,2,4-triazolo[4,3-b]pyridazin | 276°C (Zers.) | HCl | A |
| 14. | 3-Amino-6-(2-chlorphenyl)-1,2,4-triazolo[4,3-b]pyridazin | 258°C (Zers.) | HCl | A |
| 15. | 3-Amino-6-(3-fluorphenyl)-1,2,4-triazolo[4,3-b]pyridazin | 234°C (Zers.) | HCl | A |
| 16. | 3-Amino-6-(4-ethylphenyl)-1,2,4-triazolo[4,3-b]pyridazin | 263°C (Zers.) | HCl | B |
| 17. | 3-Amino-6-(4-isopropylphenyl)-1,2,4-triazolo[4,3-b]pyridazin | 253°C (Zers.) | HCl | D |
| 18. | 3-Amino-6-(4-tert. butylphenyl)-1,2,4-triazolo[4,3-b]pyridazin | 272°C (Zers.) | HCl | D |
| 19. | 3-Amino-6-(4-cyclohexylphenyl)-1,2,4-triazolo[4,3-b]pyridazin | 259°C (Zers.) | HCl | A |
| 20. | 3-Amino-6-(4-biphenylyl)-1,2,4-triazolo[4,3-b]pyridazin | 274°C (Zers.) | HCl | A |
| 21. | 3-Amino-6-(4-benzylphenyl)-1,2,4-triazolo[4,3-b]pyridazin | 297°C (Zers.) | HBr | C |
| 22. | 3-Amino-6-(3-methylphenyl)-1,2,4-triazolo[4,3-b]pyridazin | 290°C (Zers.) | HCl | C |
| 23. | 3-Amino-6-(4-methoxyphenyl)-1,2,4-triazolo[4,3-b]pyridazin | 275°C (Zers.) | HCl | D |
| 24. | 3-Amino-6-(3,4-dimethoxyphenyl)-1,2,4-triazolo[4,3-b]pyridazin | 259°C (Zers.) | HBr | A |
| 25. | 3-Amino-6-(4-methylthiophenyl)-1,2,4-triazolo[4,3-b]pyridazin | 316°C (Zers.) | HCl | A |
| 26. | 3-Amino-6-(4-phenoxyphenyl)-1,2,4-triazolo[4,3-b]pyridazin | 236°C | HCl | A |

| Beispiel | Verbindung | Fp. | Salz | Verf. |
|---|---|---|---|---|
| 27. | 3-Amino-6-(4-phenylthiophenyl)-1,2,4-triazolo[4,3-b]pyridazin | 274°C | HCl | A |
| 28. | 3-Amino-6-(2-thienyl)-1,2,4-triazolo[4,3-b]pyridazin | 280°C | HCl | A |
| 29. | 3-Amino-6-(5-methyl-2-thienyl)-1,2,4-triazolo[4,3-b]pyridazin | 318°C (Zers.) | HCl | A |
| 30. | 3-Amino-6-(5-chlor-2-thienyl)-1,2,4-triazolo[4,3-b]pyridazin | 306°C (Zers.) | HCl | A |
| 31. | 3-Amino-6-(5-brom-2-thienyl)-1,2,4-triazolo[4,3-b]pyridazin | 316°C (Zers.) | HCl | B |

**Patentansprüche für die Vertragsstaaten BE, CH, DE, FR, GB, IT, LI, LU, NL, SE**

1. 3-Amino-6-aryl-1,2,4-triazolo[4,3-b]-pyridazine der allgemeinen Formel I und deren Salze

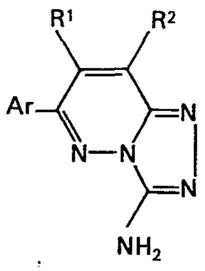

I

mit einer physiologisch verträglichen Säure, worin $R^1$ und $R^2$ gleich oder verschieden sind und Wasserstoff, Alkylgruppen mit 1-6 C-Atomen, Phenyl oder Chlor darstellen und Ar für Phenyl, Biphenyl, Phenoxyphenyl, Phenylthiophenyl, Phenylsulfinylphenyl, Phenylsulfonylphenyl, 1- oder 2-Naphthyl, 2- oder 3-Thienyl, 2-Furyl, 2-Pyrrolyl, 1-Methyl-2-pyrrolyl, 2-, 3- oder 4-Pyridyl steht, wobei diese Reste gegebenenfalls durch Fluor, Chlor, Brom, Jod, Alkylgruppen mit 1-6 C-Atomen, Cycloalkylgruppen mit 3-8 C-Atomen, Phenylalkylgruppen mit 1-4 Alkyl-C-Atomen, Alkoxy- oder Alkylthiogruppen mit jeweils 1-6 C-Atomen, Hydroxy, Nitro, Cyano, Trifluoromethyl, Carboxygruppen, deren Ester mit $C_1$-$C_6$-Alkoholen, Aminocarbonyl, Amino, Acetamino, Alkoxycarbonylamino mit 1-6 C-Atomen im Alkylrest ein-, zwei-, drei-, vier- oder fünffach substituiert sein können.

2. Verbindungen gemäss Anspruch 1, worin $R^1$ und $R^2$ gleich oder verschieden sind und Wasserstoff, Methyl, Ethyl, Phenyl oder Chlor bedeuten und in denen Ar Phenyl, Biphenyl, 2- oder 3-Thienyl, 2-Furyl, 2-, 3- oder 4-Pyridyl bedeutet, die gegebenenfalls durch ein, zwei oder drei Fluor, Chlor, Brom, Alkylgruppen mit 1-6 C-Atomen, Cycloalkylgruppen mit 3-6 C-Atomen oder Trifluormethyl substituiert sein können.

3. Verfahren zur Herstellung von Verbindungen der Formel I in Anspruch 1, dadurch gekennzeichnet, dass man

a) ein Aryl-hydrazinopyridazin der Formel II

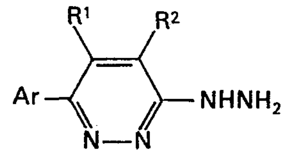

II

worin $R^1$, $R^2$ und Ar die zur Formel I angegebenen Bedeutungen haben, mit Chlorcyan, Bromcyan, O-Methylisoharnstoff oder S-Methylisothioharnstoff, Guanidin oder deren Salze, Chloroformamidinhydrochlorid oder Cyanamid als Cyclisierungsreagenz umsetzt, oder

b) ein Aryl-hydrazinopyridazin der Formel II mit einem N-($R^3$-Oxy)-carbonyl-O-methylisoharnstoff zu einer Verbindung der Formel III

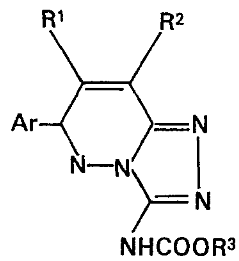

III

worin Ar, $R^1$ und $R^2$ die zur Formel I angegebenen Bedeutungen haben und $R^3$ Alkyl mit 1-10 C-Atomen, Benzyl oder Phenyl bedeutet, umsetzt und anschliessend die so erhaltene Verbindung verseift, oder

c) eine Verbindung der Formel IV, worin $R^4$ Chlor, Brom

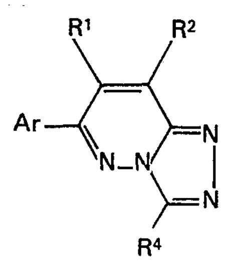

IV

oder Methylthio bedeutet, und Ar, $R^1$ und $R^2$ die zur Formel I angegebenen Bedeutungen haben, mit Ammoniak umsetzt, oder

d) eine Verbindung der Formel V

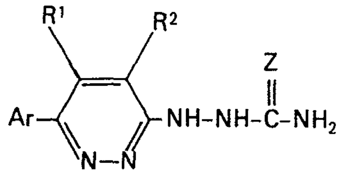

V

worin Ar, $R^1$ und $R^2$ die zur Formel I angegebenen Bedeutungen haben und Z für O, S oder NH steht, durch Erhitzen, gegebenenfalls unter Zusatz eines Kondensationsmittels, zu einer Verbindung der Formel I cyclisiert, und gegebenenfalls die so erhaltene Verbindung mit einer physiologisch verträglichen Säure in das Salz überführt.

4. Pharmazeutische Präparate mit anxiolytischer und antikonvulsiver Wirkung, gekennzeichnet durch einen Gehalt an einer Verbindung der allgemeinen Formel I in Anspruch 1.

5. Eine Verbindung der allgemeinen Formel I in Anspruch 1 oder deren Salz zur Behandlung von Erregungszuständen, Schlaflosigkeit, Krampfzuständen und vegetativer Dystonie.

**Patentanspruch für den Vertragsstaat AT**

Verfahren zur Herstellung von 3-Amino-6-aryl-1,2,4-triazolo[4,3-b]-pyridazinen der allgemeinen Formel I und von deren Salzen

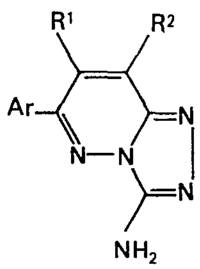

I

mit einer physiologisch verträglichen Säure, worin $R^1$ und $R^2$ gleich oder verschieden sind und Wasserstoff, Alkylgruppen mit 1-6 C-Atomen, Phenyl oder Chlor darstellen und Ar für Phenyl, Biphenyl, Phenoxyphenyl, Phenylthiophenyl, Phenylsulfinylphenyl, Phenylsulfonylphenyl, 1- oder 2-Naphthyl, 2- oder 3-Thienyl, 2-Furyl, 2-Pyrrolyl, 1-Methyl-2-pyrrolyl, 2-, 3- oder 4-Pyridyl steht, wobei diese Reste gegebenenfalls durch Fluor, Chlor, Brom, Jod, Alkylgruppen mit 1-6 C-Atomen, Cycloalkylgruppen mit 3-8 C-Atomen, Phenylalkylgruppen mit 1-4 Alkyl-C-Atomen, Alkoxy- oder Alkylthiogruppen mit jeweils 1-6 C-Atomen, Hydroxy, Nitro, Cyano, Trifluormethyl, Carboxygruppen, deren Ester mit $C_1$-$C_6$-Alkoholen, Aminocarbonyl, Amino, Acetamino, Alkoxycarbonylamino mit 1-6 C-Atomen im Alkylrest ein-, zwei-, drei-, vier- oder fünffach substituiert sein können, dadurch gekennzeichnet, dass man

a) ein Aryl-hydrazinopyridazin der Formel II

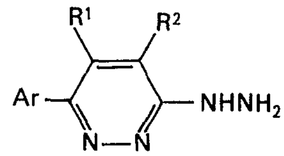

II

worin $R^1$, und $R^2$ und Ar die zu Formel I angegebenen Bedeutungen haben, mit Chlorcyan, Bromcyan, O-Methylisoharnstoff oder S-Methylisothioharnstoff, Guanidin oder deren Salzen, Chlorformamidinhydrochlorid oder Cyanamid als Cyclisierungsreagenz umsetzt, oder

b) ein Aryl-hydrazinopyridazin der Formel II mit einem N-($R^3$-Oxy)-carbonyl-O-methylisoharnstoff zu einer Verbindung der Formel III

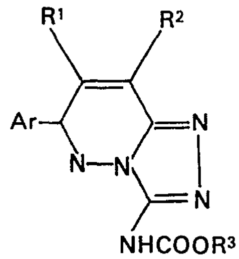

III

worin Ar, $R^1$ und $R^2$ die zu Formel I angegebenen Bedeutungen haben und $R^3$ Alkyl mit 1-10 C-Atomen, Benzyl oder Phenyl bedeutet, umsetzt und anschliessend die so erhaltene Verbindung verseift, oder

c) eine Verbindung der Formel IV, worin $R^4$ Chlor, Brom

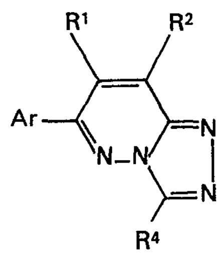

IV

oder Methylthio bedeutet, und Ar, $R^1$ und $R^2$ die zu Formel I angegebenen Bedeutungen haben, mit Ammoniak umsetzt, oder

d) eine Verbindung der Formel V

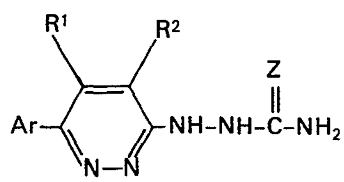

V

worin Ar, $R^1$ und $R^2$ die zu Formel I angegebene Bedeutungen haben und Z für O, S oder NH steht, durch Erhitzen, gegebenenfalls unter Zusatz eines Kondensationsmittels, zu einer Verbindung

der Formel I cyclisiert, und die so erhaltene Verbindung gegebenenfalls mit einer physiologisch verträglichen Säure in das Salz überführt.

**Claims for the Contracting States: BE, CH, DE, FR, GB, IT, LI, LU, NL, SE**

1. Amino-6-aryl-1,2,4-triazolo[4,3-b]pyridazines of the general formula I

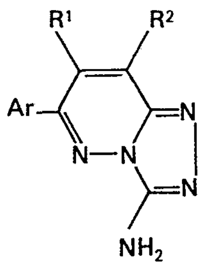

I

and their salts with a physiologically tolerated acid, wherein $R^1$ und $R^2$ are identical or different and represent hydrogen, alkyl groups having 1-6 carbon atoms, phenyl or chlorine and Ar represents phenyl, biphenyl, phenoxyphenyl, phenylthiophenyl, phenylsulfinylphenyl, phenylsulfonylphenyl, 1- or 2-naphtyl, 2- or 3-thienyl, 2-furyl, 2-pyrrolyl, 1-methyl-2-pyrrolyl, 2-, 3- or 4-pyridyl, which radicals can optionally be substituted by one, two, three, four or five fluorine, chlorine, bromine, iodine, alkyl groups having 1-6 carbon atoms, cycloalkyl groups having 3-8 carbon atoms, phenylalkyl groups having 1-4 alkyl carbon atoms, alkoxy or alkylthio groups each having 1-6 carbon atoms, hydroxyl, nitro, cyano, trifluormethyl, carboxyl groups, their esters with $C_1$-$C_6$-alcohols, aminocarbonyl, amino, acetamino or alkoxycarbonylamino having 1-6 carbon atoms in the alkyl radical.

2. Compound as claimed in claim 1, wherein $R^1$ and $R^2$ are identical or different and denote hydrogen, methyl, ethyl, phenyl or chlorine and in which Ar denotes phenyl, biphenyl, 2- or 3-thienyl, 2-furyl, 2-, 3- or 4-pyridyl which can optionally be substituted by one, two or three fluorine, chlorine, bromine, alkyl groups having 1-6 carbon atoms, cycloalkyl groups having 3-6 carbon atoms or trifluoromethyl.

3. Process for the preparation of compounds of the formula I in claim 1, which comprise

a) reacting an arylhydrazinopyridazine of the formula II

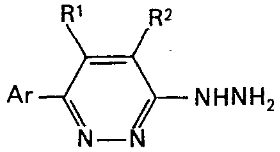

II

wherein $R^1$, $R^2$ and Ar have the meanings indicated for formula I, with cyanogen chloride, cyanogen bromide, O-methylisourea or S-methylisothiourea, guanidine or their salts, chloroformamidine hydrochloride or cyanamide as cyclization reagent, or

b) reacting an arylhydrazinopyridazine of the formula II with a N-($R^3$-oxy)carbonyl-O-methylisourea, to give a compound of the formula III

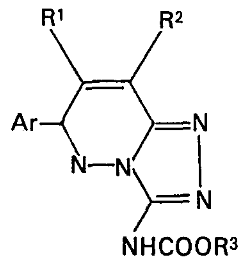

III

wherein Ar, $R^1$ and $R^2$ have the meanings indicated for formula I and $R^3$ denotes alkyl having 1-10 carbon atoms, benzyl or phenyl, and then hydrolyzing the compound thus obtained, or

c) reacting a compound of the formula IV

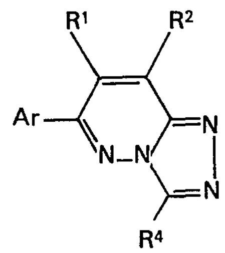

IV

wherein $R^4$ denotes chlorine, bromine or methylthio, and Ar, $R^1$ and $R^2$ have the meanings indicated for formula I, with ammonia or

d) cyclizing a compound of the formula V

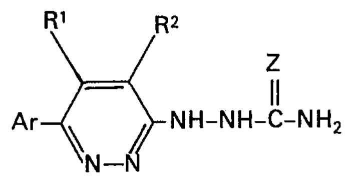

V

wherein Ar, $R^1$ and $R^2$ have the meanings indicated for formula I and Z represents O, S or NH, by heating, optionally with the addition of a condensing agent, to give a compound of the formula I, and optionally converting the compound thus obtained into the salt with a physiologically tolerated acid.

4. Pharmaceutical preparations with anxiolytic and anticonvulsive effects containing a compound of the general formula I in claim 1.

5. A compound of the general formula I in claim 1 or a salt thereof for the treatment of states of agitation, insomnia, convulsive states and neurodystonia.

**Claim for the Contracting State: AT**

1. Processes for the preparation of 3-amino-6-aryl-1,2,4-triazolo[4,3-b]pyridazines of the general formula I

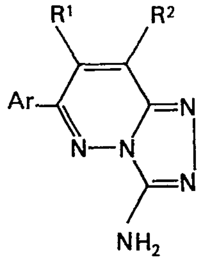

I

and of their salts with a physiologically tolerated acid, wherein $R^1$ and $R^2$ are identical or different and represent hydrogen, alkyl groups having 1-6 carbon atoms, phenyl or chlorine and Ar represents phenyl, biphenyl, phenoxyphenyl, phenylthiophenyl, phenylsulfinylphenyl, phenylsulfonylphenyl, 1- or 2-naphtyl, 2- or 3-thienyl, 2-furyl, 2-pyrrolyl, 1-methyl-2-pyrrolyl, 2-, 3- or 4-pyridyl, which radicals can optionally be substituted by one, two, three, four or five fluorine, chlorine, bromine, iodine, alkyl groups having 1-6 carbon atoms, cycloalkyl groups having 3-8 carbon atoms, phenylalkyl groups having 1-4 alkyl carbon atoms, alkoxy or alkylthio groups each having 1-6 carbon atoms, hydroxyl, nitro, cyano, trifluormethyl, carboxyl groups, their esters with $C_1$-$C_6$-alcohols, aminocarbonyl, amino, acetamino or alkoxycarbonylamino having 1-6 carbon atoms in the alkyl radical which comprise

a) reacting an arylhydrazinopyridazine of the formula II

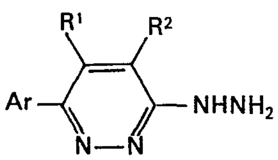

II

wherein $R^1$, $R^2$ and Ar have the meanings indicated for formula I, with cyanogen chloride, cyanogen bromide, O-methylisourea or S-methylisothiourea, guanidine or their salts, chloroformamidine hydrochloride or cyanamide as cyclization reagent, or

b) reacting an arylhydrazinopyridazine of the formula II with a N-($R^3$-oxy)carbonyl-O-methylisourea, to give a compound of the formula III

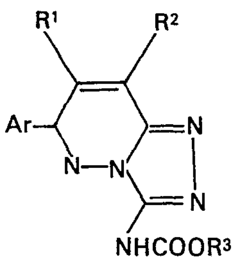

III

wherein Ar, $R^1$ and $R^2$ have the meanings indicated for formula I and $R^3$ denotes alkyl having 1-10 carbon atoms, benzyl or phenyl, and then hydrolyzing the compound thus obtained, or

c) reacting a compound of the formula IV

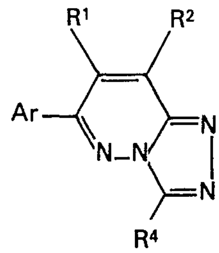

IV

wherein $R^4$ denotes chlorine, bromine or methylthio, and Ar, $R^1$ and $R^2$ have the meanings indicated for formula I, with ammonia or

d) cyclizing a compound of the formula V

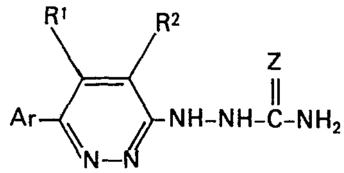

V

wherein Ar, $R^1$ and $R^2$ have the meanings indicated for formula I and Z represents O, S or NH, by heating, optionally with the addition of a condensing agent, to give a compound of the formula I, and optionally converting the compound thus obtained into the salt with a physiologically tolerated acid.

**Revendications pour les Etats contractants: BE, CH, DE, FR, GB, IT, LI, LU, NL, SE**

1. 3-amino-6-aryl-1,2,4-triazolo[4,3,b]pyridazines de formule générale I et leurs sels, avec un acide physiologiquement acceptable,

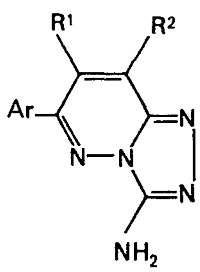

I

dans laquelle formule $R^1$ et $R^2$ sont identiques ou différents et représentent l'hydrogène, des groupes alkyle ayant de 1 à 6 atomes de carbone, phényle ou le chlore, et Ar représente un groupe phényle, biphényle, phénoxyphényle, phénylthiophényle, phénylsulfinylphényle, phénylsulfonylphényle,1- ou 2-naphtyle, 2- ou 3-thiényle, 2-furyle, 2-pyrrolyle, 1-méthyl-2-pyrrolyle 2-, 3- ou 4-pyridyle, ces groupes pouvant être éventuellement substitués une, deux, trois, quatre ou cinq fois par le fluor, le chlore, le brome, l'iode, des groupes alkyle ayant de 1 à 6 atomes de carbone,

des groupes cycloalkyle ayant de 3 à 8 atomes de carbone, des groupes phénylalkyle ayant de 1 à 4 atomes de carbone dans la partie alkyle, des groupes alcoxy- ou alkylthio ayant chacun de 1 à 6 atomes de carbone, des groupes hydroxy, nitro, cyano, trifluorométhyle, carboxy, leurs esters avec des alcools en $C_1$–$C_6$, des groupes aminocarbonyle, amino, acétamino, alcoxycarbonylamino ayant de 1 à 6 atomes de carbone dans la partie alkyle.

2. Composés selon la revendication 1, dans lesquels $R^1$ et $R^2$ sont identiques ou différents et représentent l'hydrogène, des groupes méthyle, éthyle, phényle ou le chlore et dans lesquels Ar représente un groupe phényle, biphényle, 2- ou 3-thiényle, 2-furyle, 2-, 3- ou 4-pyridyle, qui peuvent être éventuellement substitués par un, deux ou trois atomes de fluor, de chlore ou de brome ou par un, deux ou trois groupes alkyle ayant de 1 à 6 atomes de carbone, cycloalkyle ayant de 3 à 6 atomes de carbone ou trifluorométhyle.

3. Procédé pour la préparation de composés de formule I selon la revendication 1, caractérisé en ce que:

a) on fait réagir une aryl-hydrazinopyridazine de formule II

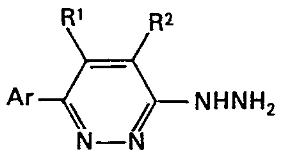

II

dans laquelle $R^1$, $R^2$ et Ar ont les significations indiquées pour la formule I, avec le chlorure de cyanogène, le bromure de cyanogène, l'O-méthylisourée ou la S-méthylisothiourée, la guanidine ou ses sels, le chlorhydrate de chloroformamidine ou le cyanamide comme agent de cyclisation, ou

b) on fait réagir une aryl-hydrazinopyridazine de formule II avec une N-($R^3$-oxy)-carbonyl-O-méthylisourée pour obtenir un composé de formule III

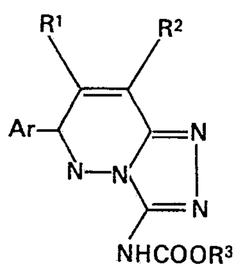

III

dans laquelle Ar, $R^1$ et $R^2$ ont les significations indiquées pour la formule I et $R^3$ représente un groupe alkyle ayant de 1 à 10 atomes de carbone, benzyle ou phényle, puis on saponifie le composé ainsi obtenu, ou

c) on fait réagir un composé de formule IV dans lequel $R^4$ représente le chlore, le brome ou un groupe méthylthio et Ar, $R^1$ et $R^2$ ont les significations indiquées pour la formule I,

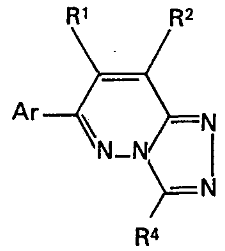

IV

avec l'ammoniac ou

d) on cyclise en un composé de formule I un composé de formule V

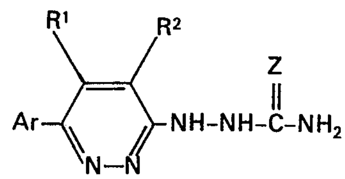

V

dans laquelle Ar, $R^1$ et $R^2$ ont les significations indiquées pour la formule I et Z représente O, S ou NH, par chauffage, éventuellement en présence d'un agent de condensation, et éventuellement on convertit le composé ainsi obtenu en un sel au moyen d'un acide physiologiquement acceptable.

4. Composition pharmaceutique avec une action anxiolytique et anticonvulsivante, caractérisée par une teneur en un composé de formule générale I selon la revendication 1.

5. Composé de formule générale I selon la revendication 1 ou son sel pour le traitement des états d'excitation, l'insomnie, l'apparition de crampes et la dystonie végétative.

**Revendication pour l'Etat contractant: AT**

Procédé pour la préparation de 3-amino-6-aryl-1,2,4-triazolo[4,3,b]-pyridazines de formule générale I et de leurs sels avec un acide physiologiquement acceptable,

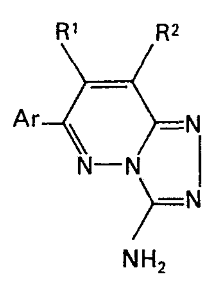

I

dans laquelle formule $R^1$ et $R^2$ sont identiques ou différents et représentent l'hydrogène, des groupes alkyle ayant de 1 à 6 atomes de carbone, des groupes phényle ou le chlore et Ar représente un groupe phényle, biphényle, phénoxyphényle, phénylthiophényle, phénylsulfinylphényle, phénylsulfonylphényle, 1- ou 2-naphtyle, 2- ou 3-thiényle, 2-furyle, 2-pyrrolyle, 1-méthyl-2-pyrrolyle, 2-, 3- ou 4-pyridyle, ces groupes pouvant éventuellement être une, deux, trois, quatre ou cinq fois substitués par le fluor, le chlore, le brome, l'iode, des groupes alkyle ayant de 1 à 6 atomes de carbone, des groupes cycloalkyle ayant de 3 à 8 atomes de carbone, des groupes phénylalkyle dont la partie alkyle a de 1 à 4 atomes de carbone, des groupes alcoxy ou alkylthio ayant chacun de 1 à 6 atomes de carbone, des groupes hydroxy, nitro, cyano, trifluorométhyle, carboxy, leurs esters avec des alcools en $C_1$-$C_6$, des groupes aminocarbonyle, amino, acétamino, alcoxycarbonylamino ayant de 1 à 6 atomes de carbone dans la partie alkyle, lequel procédé est caractérisé en ce que:

a) on fait réagir une aryl-hydrazinopyridazine de formule II

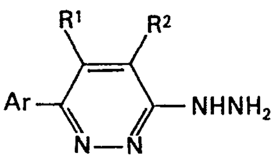

II

dans laquelle $R^1$, $R^2$ et Ar ont les significations indiquées pour la formule I, avec le chlorure de cyanogène, le bromure de cyanogène, l'O-méthylisourée ou la S-méthylisothiourée, la guanidine ou ses sels, le chlorhydrate de chloroformamidine ou le cyanamide comme agent de cyclisation, ou

b) on fait réagir une aryl-hydrazinopyridazine de formule II avec une N-($R^3$-oxy)-carbonyl-O-méthylisourée pour obtenir un composé de formule III

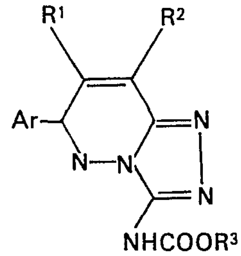

III

dans laquelle Ar, $R^1$ et $R^2$ ont les significations indiquées pour la formule I et $R^3$ représente un groupe alkyle ayant de 1 à 10 atomes de carbone, benzyle ou phényle, puis on saponifie le composé ainsi obtenu, ou

c) on fait réagir un composé de formule IV, dans lequel $R^4$ représente le chlore, le brome ou un groupe méthylthio et Ar, $R^1$ et $R^2$ ont les significations indiquées pour la formule I.

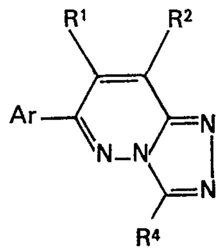

IV

avec l'ammoniac ou

d) on cyclise en un composé de formule I un composé de formule V

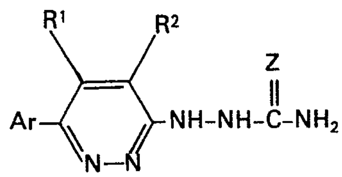

V

dans laquelle Ar, $R^1$ et $R^2$ ont les significations indiquées pour la formule I et Z représente O, S ou NH, par chauffage, éventuellement en présence d'un agent de condensation, et on convertit éventuellement le composé ainsi obtenu en le sel au moyen d'un acide physiologiquement acceptable.